# EUROPEAN PATENT APPLICATION

(11) **EP 1 632 241 A1**
(43) Date of publication of application: **08.03.2006**
(21) Application number: 04733114.5
(22) Date of filing: 14.05.2004
(51) Int. Cl.: A61K 38/00, A61K 45/00, A61K 31/203, A61K 47/48, A61P 1/00, A61P 1/04, A61P 1/16, A61P 1/18, A61P 3/10, A61P 7/02, A61P 9/00, A61P 9/04, A61P 9/08, A61P 9/10, A61P 11/00, A61P 11/06, A61P 11/08, A61P 13/12

(54) **PREVENTIVE AND/OR REMEDY FOR DISEASES ACCOMPANIED BY TISSUE DESTRUCTION**

(30) Priority: 16.05.2003 JP 2003139604
(71) Applicant: KYOWA HAKKO KOGYO CO., LTD., Chiyoda-ku, Tokyo 100-8185 (JP)
(72) Inventor: SAKURADA, Kazuhiro, Kanawaga 225-0016 (JP); YAMADA, Yoji c/o BioFrontier Labs., Machida-shi, Tokyo 194-8533 (JP); ANDO, Hiroshi c/o BioFrontier Labs., Tokyo 194-8533 (JP); SATO, Hidetaka c/o BioFrontier Labs., Machida-shi, Tokyo 194-8533 (JP); YOKOYAMA, Hiromi c/o BioFrontier Labs., Machida-shi, Tokyo 194-8533 (JP); ISHIHARA, Masahiko c/o Head Office, Chiyoda-ku,Tokyo 100-8185 (JP); MIKI, Ichiro c/o Pharma.Res.Center, Nagaizumi-cho, Sunto-gun,Shizuoka 411-8731 (JP); WATANABE, Akiko Pharma.Res.Center, Nagazumi-cho, Sunto-gun, Shizuoka 411-8731 (JP)
(74) Representative: Vossius & Partner
(86) International application number: PCT/JP2004/006895
(87) International publication number: WO 2004/100972

(57) **Abstract**

The present invention relates to an agent for preventing and/or treating diseases accompanied by tissue disruption, which comprises a polypeptide having granulocyte colony-stimulating factor activity as an active ingredient, and a medicament for mobilizing a multipotent stem cell from a tissue into peripheral blood, which comprises a polypeptide having granulocyte colony-stimulating factor activity as an active ingredient.

## Description

### TECHNICAL FIELD

The present invention relates to an agent for preventing and/or treating diseases accompanied by tissue disruption, which comprises a polypeptide having granulocyte colony-stimulating factor (hereinafter referred to as "G-CSF") activity as an active ingredient, and a medicament for mobilizing a multipotent stem cell from a tissue into peripheral blood, which comprises a polypeptide having G-CSF activity as an active ingredient.

### BACKGROUND ART

G-CSF is a polypeptide which proliferates or differentiates neutrophilic granulocyte precursor cells and activates mature neutrophils. G-CSF is mainly used for accelerating increase of neutrophils in the case of bone marrow transplantation, neutropenia caused by chemotherapy for cancer, myelodysplastic syndrome, hypoplastic anemia, congenital or sudden neutropenia, human immunodeficiency virus (HIV) infection and the like (*Shorei ni Manabu G-CSF no Rinsho (Clinic of G-CSF Learned from Cases),* edited by Yasuo Ikeda, published by Iyaku Journal, Osaka, pp.64-92 (1999)). It is recently reported that G-CSF increases stem cells in peripheral blood (*Shorei ni Manabu G-CSF no Rinsho (Clinic of G-CSF Learned from Cases),* edited by Yasuo Ikeda, published by Iyaku Journal, Osaka, pp.139-168 (1999)). As the stem cells, hematopoietic stem cells, somatic stem cells (tissue stem cells) and the like are known.

It has been shown that various subtypes are included in the hematopoietic stem cells which are transferred into peripheral blood by G-CSF (*Blood,* 84, 2795-2801 (1994)). Hematopoietic factors including G-CSF, cell adhesion molecules, chemokine, metalloprotease and the like mutually relate to transfer of stem cells from bone marrow into peripheral blood (*Experimental Hematology*, 30, 973-981 (2002)). A chemokine receptor CXCR4 and its ligand CXCL12 (stromal cell-derived factor 1, SDF-1) relate to transfer of stem cells from bone marrow into peripheral blood by G-CSF, and cyclophosphamide also has equivalent physiological activity other than G-CSF (*The Journal of Clinical Investigation,* 111, 187-196 (2003)).

It is known that a chemokine CXCR4 inhibitor AMD-3100 (U.S. Patent 5,612,478) also has activity to transfer hematopoietic stem cells from bone marrow into peripheral blood (American Society of Hematology, Philadelphia, USA, June 6-10, 2002).

As one of the methods for mobilizing hematopoietic stem cells into peripheral blood, a method for administering a granulocyte colony-stimulating factor (G-CSF) is known (*Blood,* 90, 903-908 (1997)).

Furthermore, it has been reported that, when CD34-positive cells isolated from cells to be mobilized by G-CSF into human peripheral blood are transplanted into a myocardial infarction model, angiogenesis is caused and cardiac function is improved *(Nature Medicine,* 7, 430-436 (2001), WO2001/94420).

Also, since regeneration of heart muscle and blood vessel is caused in the infarction region by administering G-CSF and a stem cell factor (SCF) to a mouse before causing myocardial infarction, it is considered that hematopoietic stem cells are flowed out by G-CSF into peripheral blood and that the infarction heart muscle is regenerated (*Proc. Natl. Acad Sci. USA,* 98, 10344-10349 (2001)).

Corti *et al*. have reported that a bone marrow-derived neuron increases in the brain by administering G-CSF and SCF to a mouse (*Experimental Neurology,* 173(2), 443-452 (2002)).

Furthermore, in human, when cells mobilized to peripheral blood by the administration of G-CSF are transplanted, donor-derived cells have been detected in the liver, digestive tract epithelium and skin (*N. England J. Med.,* 346, 738-746 (2002)) and buccal epithelium (*Lancet*, 361, 1084-1088 (2003)) of human recipients.

However, in tissues other than the hematopoietic systems, it has not been found what kind of stem cells mobilized by administration of G-CSF causes differentiation of tissue. Furthermore, it has not been found whether or not diseases other than blood cell systems and circulatory organ systems can be treated by administration of G-CSF or transplantation of stem cells mobilized by G-CSF.

Serious diseases accompanied by tissue disruption of the lungs include pulmonary emphysema, chronic bronchitis, chronic obstructive pulmonary disease (hereinafter referred to as "COPD"), cystic fibrosis, sudden interstitial pneumonia (pulmonary fibrosis), diffuse pulmonary fibrosis, tuberculosis, asthma and the like. Particularly, disruption of alveolar is prominent in pulmonary emphysema and COPD (Shoji Kudo, *Kokyuki Shikkan no Chiryo to Kango (Treatment and Nursing of Respiratory Organ Diseases),* published by Nankodo on March, 2002, and *Shorei ni Manabu G-CSF no Rinsho (Clinic of G-CSF Learned from Cases),* edited by Yasuo Ikeda, published by Iyaku Journal, Osaka, pp.64-92 (1999)). Pulmonary emphysema, chronic bronchitis and COPD are diseases in which inflammatory lesion is differentiated in bronchus, bronchiole or alveolus, and difficulty of breathing is caused when disruption of alveoli progresses. Since there is no sufficient method for treating tissue disruption of the lungs at present, the development of highly effective preventive agent, therapeutic agent and therapeutic method has been desired.

It is known that retinoic acid relates to maturation of the lungs of fetal period. It has been reported that, when retinoic acid was administered to a rat in which alveoli were disrupted by elastase, alveoli were repaired *(Nature Medicine,* 3, 675-677 (1997)). It has also been reported that a retinoic acid derivative RO444753 (*Journal of Medicinal Chemistry*, 31, 2182-2192 (1988)) has similar therapeutic effect on pulmonary emphysema (*American Journal of Respiratory and Critical Care Medicine,* 165(8), A825 (2002)). Although various cases using retinoic acid for the treatment of disease accompanied by disruption of alveoli have been reported, high effect has not been obtained by any of them.

In recent years, as a result of tests of stem cell transplantation, it has been found that alveoli are differentiated frequently from stem cells (*Cell,* 105, 369-377 (2001)).

Chronic hepatic diseases including hepatic cirrhosis are the forth ranking of the cause of death in an age group of 30 years to 64 years. Also, included in the malignant neoplasm (cancer) which is the first ranking of the cause of death are 22,900 dead males (the third next to lung cancer and gastric cancer) and 9,400 dead females (the fourth next to gastric cancer, lung cancer and colon cancer) due to the cancer of liver caused by hepatic diseases such as hepatic cirrhosis. The numbers of the persons infected with hepatitis virus, which is the main cause of hepatitis, are 1,500,000 persons in the case of hepatitis B and 2,500,000 persons in the case of hepatitis C. Interferon is administered as the antiviral agent for such viral hepatitis, but its transition into hepatic cirrhosis cannot be stopped completely. Furthermore, according to investigation carried out by the Ministry of Health and Welfare in Japan, about 2,200,000 people are drinking 5 gou (corresponding to 0.9 liter) or more of alcohol as refined sake every day at present. Patients of hepatic diseases such as fatty liver, alcoholic hepatitis and hepatic cirrhosis are found frequently in these problematic drinkers. About 300,000 per year of people are newly developing hepatic cirrhosis including viral and alcoholic ones. However, at present, there is absolutely no method for treating hepatic cirrhosis. Recently, it has been reported that the liver function is improved through the disappearance of hepatic fibers and regeneration of hepatocytes by transplanting bone marrow cells into a carbon tetrachloride hepatopathy model which can be used as a model of alcoholic hepatitis (The Second Meeting of the Japanese Society for Regenerative Medicine, Kobe, March 11-12, 2003).

Since the number of patients requiring dialysis is now close to 170,000 in Japan, a kidney regeneration agent for completely curing renal insufficiency is in markedly large clinical needs, but there is absolutely no effective therapeutic method other than kidney transplantation. Recently, it has been reported that renal glomerulus and renal tubular epithelium are differentiated from bone marrow cells, and it has been found that kidney tissue is differentiated from the bone marrow stem cell (*Kidney International*, 62, 1285-1290 (2002)).

### DISCLOSURE OF THE INVENTION

An object of the present invention is to provide an agent for preventing and/or treating diseases accompanied by tissue disruption, which comprises a polypeptide having G-CSF activity as an active ingredient, or a medicament for mobilizing a multipotent stem cell from a tissue into peripheral blood, which comprises a polypeptide having G-CSF activity as an active ingredient.

The present invention relates to the following (1) to (35).
(1) An agent for preventing and/or treating diseases accompanied by tissue disruption, which comprises a polypeptide having granulocyte colony-stimulating factor activity as an active ingredient.
(2) The agent according to (1), wherein the polypeptide comprises the amino acid sequence represented by SEQ ID NO:1.
(3) The agent according to (1), wherein the polypeptide consists of an amino acid sequence in which at least one amino acid residue in the amino acid sequence represented by SEQ ID NO: 1 is deleted, substituted and/or added, and has granulocyte colony-stimulating factor activity.
(4) The agent according to (1), wherein the polypeptide consists of an amino acid sequence having a homology of 80% or more with the amino acid sequence represented by SEQ ID NO: 1, and has granulocyte colony-stimulating factor activity.
(5) The agent according to any one of (1) to (4), wherein the polypeptide is a chemically modified polypeptide having granulocyte colony-stimulating factor activity.
(6) The agent according to (5), wherein the polypeptide is modified with polyalkylene glycol.
(7) An agent for preventing and/or treating diseases accompanied by tissue disruption, which comprises (a) a polypeptide having granulocyte colony-stimulating factor activity and (b) retinoic acid or a retinoic acid derivative, wherein (a) and (b) are administered simultaneously, separately by keeping a period or as a single medicament comprising both of (a) and (b).
(8) An agent for preventing and/or treating diseases accompanied by tissue disruption, which comprises (a) a polypeptide having granulocyte colony-stimulating factor activity and (b) a CXCR4 inhibitor, wherein (a) and (b) are administered simultaneously, separately by keeping a period or as a single medicament comprising both of (a) and (b).
(9) The agent according to (8), wherein the CXCR4 inhibitor is AMD-3100 or a derivative thereof.
(10) The agent according to any one of (1) to (9), wherein the disease accompanied by tissue disruption is selected from the group consisting of nervous diseases, circulatory organ system diseases, hepatic diseases, pancreatic diseases, digestive tract system diseases, renal diseases, skin diseases and lung diseases.
(11) The agent according to (10), wherein the nervous disease is selected from the group consisting of cerebral infarction, cerebrovascular accidents, Parkinson disease, Alzheimer disease, Huntington chorea, spinal cord injury, depression and manic-depressive psychosis.
(12) The agent according to (10), wherein the circulatory organ system disease is selected from the group consisting of obstructive vascular disease, myocardial infarction, cardiac failure and coronary artery disease.
(13) The agent according to (10), wherein the hepatic disease is selected from the group consisting of hepatitis B, hepatitis C, alcoholic hepatitis, hepatic cirrhosis and hepatic insufficiency.
(14) The agent according to (10), wherein the pancreatic disease is selected from the group consisting of diabetes mellitus and pancreatitis.
(15) The agent according to (10), wherein the digestive tract system disease is selected from the group consisting of Crohn disease and ulcerative colitis.
(16) The agent according to (10), wherein the renal disease is selected from the group consisting of IgA nephropathy, glomerular nephritis and renal insufficiency.
(17) The agent according to (10), wherein the skin disease is selected from the group consisting of decubitus, burn injury, suture wound, lacerated wound, incision wound, bite wound, dermatitis, cicatricial keloid, keloid, diabetic ulcer, arterial ulcer and venous ulcer.
(18) The agent according to (10), wherein the lung disease is selected from the group consisting of pulmonary emphysema, chronic bronchitis, chronic obstructive pulmonary disease, cystic fibrosis, sudden interstitial pneumonia (pulmonary fibrosis), diffuse pulmonary fibrosis, tuberculosis or asthma.
(19) A medicament for mobilizing a multipotent stem cell from a tissue into peripheral blood, which comprises a polypeptide having granulocyte colony-stimulating factor activity as an active ingredient.
(20) The medicament according to (19), wherein the polypeptide comprises the amino acid sequence represented by SEQ ID NO:1.
(21) The medicament according to (19), wherein the polypeptide consists of an amino acid sequence in which at least one amino acid residue in the amino acid sequence represented by SEQ ID NO:1 is deleted, substituted and/or added, and has granulocyte colony-stimulating factor activity.
(22) The medicament according to (19), wherein the polypeptide consists of an amino acid sequence having a homology of 80% or more with the amino acid sequence represented by SEQ ID NO: 1, and has granulocyte colony-stimulating factor activity.
(23) The medicament according to any one of (19) to (22), wherein the polypeptide is a chemically modified polypeptide having granulocyte colony-stimulating factor activity.
(24) The medicament according to (23), wherein the polypeptide is modified with polyalkylene glycol.
(25) A medicament for mobilizing a multipotent stem cell from a tissue into peripheral blood, which comprises (a) a polypeptide having granulocyte colony-stimulating factor activity and (b) retinoic acid or a retinoic acid derivative, wherein (a) and (b) are administered simultaneously, separately by keeping a period or as a single medicament comprising both of (a) and (b).
(26) A medicament for mobilizing a multipotent stem cell from a tissue into peripheral blood, which comprises (a) a polypeptide having granulocyte colony-stimulating factor activity and (b) a CXCR4 inhibitor, wherein (a) and (b) are administered simultaneously, separately by keeping a period or as a single medicament comprising both of (a) and (b).
(27) The medicament according to (26), wherein the CXCR4 inhibitor is AMD-3100 or a derivative thereof
(28) A method for preventing and/or treating diseases accompanied by tissue disruption, which comprises administering a polypeptide having granulocyte colony-stimulating factor activity.
(29) A method for preventing and/or treating diseases accompanied by tissue disruption, which comprises administering (a) a polypeptide having granulocyte colony-stimulating factor activity and (b) retinoic acid or a retinoic acid derivative simultaneously or separately by keeping a period.
(30) A method for preventing and/or treating diseases accompanied by tissue disruption, which comprises administering (a) a polypeptide having granulocyte colony-stimulating factor activity and (b) a CXCR4 inhibitor simultaneously or separately by keeping a period.
(31) A method for mobilizing a multipotent stem cell from a tissue into peripheral blood, which comprises administering a polypeptide having granulocyte colony-stimulating factor activity.
(32) A method for mobilizing a multipotent stem cell from a tissue into peripheral blood, which comprises administering (a) a polypeptide having granulocyte colony-stimulating factor activity and (b) retinoic acid or a retinoic acid derivative simultaneously or separately by keeping a period.
(33) A method for mobilizing a multipotent stem cell from a tissue into peripheral blood, which comprises (a) a polypeptide having granulocyte colony-stimulating factor activity and (b) a CXCR4 inhibitor simultaneously or separately by keeping a period.
(34) Use of a polypeptide having granulocyte colony-stimulating factor activity for the manufacture of an agent for preventing and/or treating diseases accompanied by tissue disruption.
(35) Use of a polypeptide having granulocyte colony-stimulating factor activity for the manufacture of a medicament for mobilizing a multipotent stem cell from a tissue into peripheral blood.

### 1. Agent for preventing and/or treating diseases accompanied by tissue disruption

In the present invention, diseases accompanied by tissue disruption include nervous diseases, circulatory organ system diseases, hepatic diseases, pancreatic diseases, digestive tract system diseases, renal diseases, skin diseases, lung diseases and the like.

The nervous diseases include cerebral infarction, cerebrovascular accidents, Parkinson disease, Alzheimer disease, Huntington chorea, spinal cord injury, depression, manic-depressive psychosis and the like.

The circulatory organ system diseases include obstructive vascular disease, myocardial infarction, cardiac failure, coronary artery disease and the like.

The hepatic diseases include hepatitis B, hepatitis C, alcoholic hepatitis, hepatic cirrhosis, hepatic insufficiency and the like.

The pancreatic diseases include diabetes mellitus, pancreatitis and the like.

The digestive tract system diseases include Crohn disease, ulcerative colitis and the like.

The renal diseases include IgA nephropathy, glomerular nephritis, renal insufficiency and the like.

The skin diseases include decubitus, burn injury, suture wound, lacerated wound, incision wound, bite wound, dermatitis, cicatricial keloid, keloid, diabetic ulcer, arterial ulcer, venous ulcer and the like.

The lung diseases include pulmonary emphysema, chronic bronchitis, chronic obstructive pulmonary disease, cystic fibrosis, sudden interstitial pneumonia (pulmonary fibrosis), diffuse pulmonary fibrosis, tuberculosis, asthma and the like.

The polypeptide having G-CSF activity includes a polypeptide comprising the amino acid sequence represented by SEQ ID NO: 1, or a polypeptide which consists of an amino acid sequence in which at least one amino acid residue in the amino acid sequence represented by SEQ ID NO:1 is deleted, substituted and/or added, and has G-CSF activity.

Examples include nartograstim (trade name: Neu-up, manufactured by Kyowa Hakko Kogyo Co., Ltd.), filgrastim (trade name: Gran, manufactured by SANKYO; trade name: Granulokine, manufactured by Hoffmann-La Roche; trade name: Neupogen, manufactured by Amgen), lenograstim (trade name: Neutrogin, manufactured by Chugai Pharmaceutical; trade name: Granocyte, manufactured by Aventis), pegfilgrastim (trade name: Neulasta, manufactured by Amgen), sargramostim (trade name: Leukine, manufactured by Schering) and the like.

Also, the polypeptide having G-CSF activity includes a polypeptide having a homology of preferably 60% or more, more preferably 80% or more, still more preferably 90% or more, and most preferably 95% or more when its amino acid sequence homology with G-CSF having the amino acid sequence represented by SEQ ID NO:1 is searched by BLAST (Basic Local Alignment Search Tool). Examples of the polypeptides in which at least one amino acid residue in the amino acid sequence represented by SEQ ID NO: 1 is substituted, which has the G-CSF activity are shown in Table 1.

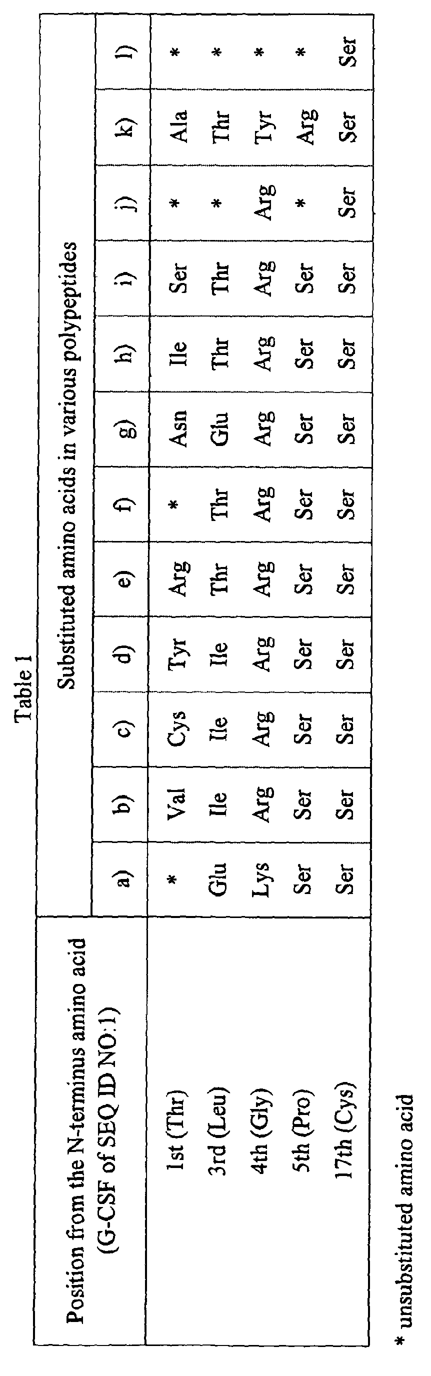

Furthermore, the polypeptide having G-CSF activity may be chemically modified.

The chemical modification method includes the method described in WO00/51626 and the like, and a polypeptide having G-CSF activity modified with, for example, polyalkylene glycol such as polyethylene glycol (PEG) is included.

The medicament which comprises the polypeptide having G-CSF activity of the present invention as an active ingredient can be administered alone as a therapeutic agent, but generally, it is preferred to provide it as a pharmaceutical preparation produced by any method known in the technical field of manufacturing pharmacy, by mixing it together with at least one pharmaceutically acceptable carriers.

It is preferred to use a route of administration which is most effective in the treatment, and examples include oral administration and parenteral administration such as buccal, airway, rectal, intramuscular, subcutaneous, intradermal and intravenous administrations. Among these, intramuscular, subcutaneous, intradermal, intravenous or airway administration is preferred.

The dosage forms include tablets, capsules, granules, injections, ointments, tapes, dry powders, inhalations such as aerosols and the like.

In the production of solid preparations such as tablets, for example, excipient such as lactose; disintegrating agents such as starch; lubricants such as magnesium stearate; binders such as hydroxypropylcellulose; surfactants such as fatty acid ester; plasticizers such as glycerol; and the like can be used.

The pharmaceutical preparation suitable for intramuscular, subcutaneous, intradermal, intravenous or airway administration includes injections, sprays and the like.

In the production of injections, for example, water, saline, plant oil such as soybean oil, a solvent, a solubilizing agent, a tonicity agent, a preservative, an antioxidant, and the like can be used.

Also, inhalations are prepared by using the polypeptide having G-CSF activity alone or together with a carrier or the like which does not irritate buccal and airway mucous membranes of the recipient and can facilitate absorption of the polypeptide having G-CSF activity by dispersing it as fine particles. The carrier includes lactose, glycerol and the like. Depending on the properties of the polypeptide having G-CSF activity and the carrier to be used, preparations such as aerosols and dry powders can be produced. Furthermore, the components exemplified as the additives of oral preparations can also be added to these parenteral preparations.

Although the dose and administration frequency vary depending on the intended therapeutic effect, administration method, treating period, age, body weight and the like, generally, it is preferred to administer in a dose of 0.01 µg/kg to 10 mg/kg per day per adult.

### 2. Combined use of the granulocyte colony-stimulating factor and other medicament

The activity of the polypeptide having G-CSF activity used in the present invention can be increased by using retinoic acid, a retinoic acid derivative or a CXCR4 inhibitor in combination.

Any retinoic acid derivatives can be used, so long as they bind to the retinoic acid receptor, and examples include retinoic acid derivatives such as retinol palmitate, retinol, retinal, 3-dehydroretinoic acid, 3-dehydroretinol, and 3-dehydroretinal; provitamin A such as α-carotene, β-carotene, γ-carotene, β-cryptoxanthin, and echinenone; and the like. Other examples include motretinide (trade name: Tasmaderm, manufactured by Hoffmann-La Roche, see US Patent 4,105,681), compounds described in WO 02/04439, Tazaroten (trade name: Tazorac, manufactured by Allergan, see EP284288), AGN-194310 and AGN-195183 (manufactured by Allergan, see WO 97/09297), retinoic acid TopiCare (trade name: Avita, manufactured by Mylan Laboratories), UAB-30 (CAS Number 205252-59-1, manufactured by UAB Research Foundation) and the like.

The CXCR4 inhibitor includes AMD-3100 and the like.

The polypeptide having G-CSF activity used in the present invention and retinoic acid, a retinoic acid derivative or a CXCR4 inhibitor can be used or administered either as a single preparation (mixed preparation) or as a combination of plural preparations, so long as respective active ingredients are contained. When they are used as a combination of plural preparations, they can be used or administered simultaneously or separately by keeping a period. Also, these pharmaceutical preparations can be used in the form of, for example, tablets, capsules, granules, injections, ointments, tapes, inhalations such as dry powders and aerosols, and the like.

A preferred dose ratio (weight/weight) of the polypeptide having G-CSF activity used in the present invention and retinoic acid, a retinoic acid derivative or a CXCR4 inhibitor can be optionally adjusted according to the combination of the retinoic acid, retinoic acid derivative or CXCR4 inhibitor and the efficacy of the retinoic acid, retinoic acid derivative or CXCR4 inhibitor. For example, they can be used at a ratio of 1/50,000 (granulocyte colony-stimulating factor/retinoic acid, retinoic acid derivative or CXCR4 inhibitor) to 100/1, preferably from 1/10,000 to 20/1, and more preferably from 1/1,000 to 10/1.

When they are administered as a combination of plural pharmaceutical preparations, for example, (a) a first component comprising the polypeptide having G-CSF activity and (b) a second component comprising retinoic acid, a retinoic acid derivative or a CXCR4 inhibitor can be separately formulated as described above to prepare a kit and respective components can be administered by using the kit simultaneously, or separately by keeping a period, to the same subject through the same route or different routes.

The kit which can be used is a kit which comprise two or more containers (e.g., vial, bag, *etc*.) and the contents, wherein materials, shapes and the like of the containers are not particularly limited so long as they are such containers that denaturation of components as the contents by external temperature and light or dissolution of chemical components from the containers do not change during storage, and which also have such a mode that the above first component and second component as the contents can be administered through separate routes (e.g., tube, *etc*.) or the same route. Examples include kits of tablets, injections, inhalations and the like.

The above pharmaceutical preparations can be produced according to the conventional method by using pharmaceutically acceptable diluents, excipients, disintegrating agents, lubricants, binders, surfactants, water, saline, plant oil solubilizing agents, tonicity agents, preservatives, antioxidants and the like.

In the production of tablets, for example, excipients such as lactose; disintegrating agents such as starch; lubricants such as magnesium stearate; binders such as hydroxypropylcellulose; surfactants such as fatty acid ester; plasticizers such as glycerol; and the like can be used.

In the production of injections, for example, water, saline, plant oil such as soybean oil, a solvent, a solubilizing agent, a tonicity agent, a preservative, an antioxidant, and the like can be used.

Also, inhalations are prepared by using the polypeptide having G-CSF activity alone or together with a carrier or the like which does not irritate buccal and airway mucous membranes of the receptor and can facilitate absorption of the polypeptide having G-CSF activity by dispersing it as fine particles. The carrier includes lactose, glycerol and the like. Furthermore, the components exemplified as the additives of oral preparations can also be added to these parenteral preparations.

Although the dose and administration frequency vary depending on the intended therapeutic effect, administration method, treating period, age, body weight and the like, generally, it is preferred to administer the polypeptide having G-CSF activity in a dose of 0.01 µg/kg to 10 mg/kg, or the retinoic acid, retinoic acid derivative or CXCR4 inhibitor in a dose of 0.1 mg/kg to 100 mg/kg, per day per adult.

### 3. Medicament for mobilizing pluripotent stem cell into peripheral blood

The agent for preventing and/or treating diseases accompanied by tissue disruption as described in the above item 1 or 2 can mobilize pluripotent stem cells into peripheral blood and regenerate injured tissue by the mobilized pluripotent stem cells.

### 4. Method for treating diseases using the medicament of the present invention

As the method for treating diseases using the agent for preventing and/or treating diseases accompanied by tissue disruption of the present invention, any method can be used such as a method in which an injured region is repaired by directly administering the agent of the present invention to the patient as described in the above item 1 or a method in which pluripotent stem cells mobilized to peripheral blood by the medicament of the above item 3 are recovered, and the recovered pluripotent stem cells are transplanted into the injured region, directly or after differentiating them into cells or tissues of interest *in vitro.*

When pluripotent stem cells or differentiated cells are used in a treatment, the pluripotent stem cells or differentiated cells are washed with saline by using, for example, Hemolite 2 Plus manufactured by Hemonetics. The apparatus used includes those in which concentration, washing and recovery of cultured cells are carried out in a completely closed system, and preferred is one capable of removing substances, such as cytokine used in the culturing and differentiation, as close as 100%. The pluripotent stem cells thus recovered or cells thus differentiated can be used in the treatment by intravenously injecting them according to the general drip infusion method or directly injecting them into the affected part.

### 5. Method for evaluating the agent for preventing and/or treating diseases accompanied by tissue disruption of the present invention

### (1) Brain or nervous system tissue

For example, according to the following method, it can be confirmed that the agent for preventing and/or treating diseases accompanied by tissue disruption of the present invention can be used for treatment of diseases accompanied by disruption of brain or nervous system tissue.

The above agent for preventing and/or treating diseases accompanied by tissue disruption of the present invention is administered to an experimental animal such as mouse, rat or monkey, and if the alleviation of symptoms accompanied by disruption is observed or a differentiated brain or nervous system cells are identified, it can be confirmed that the agent is effective for treatment of disease accompanied by disruption of a brain or nervous system tissue. Preferred as the experimental animal is an animal whose brain is damaged by ischemia, administration of 6-hydroxydopamine (6-OHDA), administration of kainic acid or the like. The route of administration of the agent for preventing and/or treating diseases accompanied by tissue disruption of the present invention includes subcutaneous administration.

For example, nervous system cells differentiated inside the brain can be detected by the following method.

After irradiating a lethal dose of radiation in advance, a bone marrow chimeric mouse transplanted with a bone marrow derived from a transgenic mouse of the same strain capable of constitutively expressing green fluorescent protein (GFP) is prepared, and the brain of the bone marrow chimeric mouse is damaged by the above method, and then the agent for preventing and/or treating diseases accompanied by tissue disruption of the present invention is administered. The differentiated nervous system cells can be identified by measuring fluorescence intensity of GFP.

### (2) Liver tissue

For example, according to the following method, it can be confirmed that the agent for preventing and/or treating diseases accompanied by tissue disruption of the present invention can be used for treatment of diseases accompanied by disruption of liver tissue.

The above agent for preventing and/or treating diseases accompanied by tissue disruption of the present invention is administered to a hepatocyte-disrupted model mouse, and if the alleviation of symptoms accompanied by disruption is observed or new cell generation in the liver is accelerated, it can be confirmed that the agent is effective for treatment of diseases accompanied by disruption of liver tissue. Model animal showing disease accompanied by disruption of hepatocytes include a model in which mainly carbon tetrachloride esterase is injected into the abdominal cavity (*American Journal of Pathology*, 161, 2003-2010 (2002)), a model in which the liver is partially excised (*Arch. Pathol*., 12, 186-202 (1931)) and the like. Furthermore, gene-modified animals having a morbid state of liver disruption include a fumarylacetate hydrolase (FAH)-deficient mouse (*Nature Medicine,* 6, 1229-1234 (2000)) and the like.

The hepatocyte disruption can be evaluated by measuring the concentration of GPT (glutamic pyruvic transaminase), GOT (glutamic oxalacetic transaminase), bilirubin, γ-GTP and the like in blood plasma or serum.

The hepatocyte differentiated in the liver can be detected by the following method.

After irradiating a lethal dose of radiation in advance, a bone marrow chimeric mouse transplanted with a bone marrow derived from a transgenic mouse of the same strain capable of constitutively expressing green fluorescent protein (GFP) is prepared, and the liver of the bone marrow chimeric mouse is damaged by the above method, and then the agent for preventing and/or treating diseases accompanied by tissue disruption of the present invention having G-CSF activity as the active ingredient is administered. The differentiated hepatocytes can be identified based on fluorescence of GFP.

### (3) Pancreatic tissue

For example, according to the following method, it can be confirmed that the agent for preventing and/or treating diseases accompanied by tissue disruption of the present invention is useful for treatment of diseases accompanied by disruption of pancreatic tissue.

The above agent for preventing and/or treating diseases accompanied by tissue disruption of the present invention is administered to a pancreatic cell-disrupted model animal, and if the alleviation of symptoms accompanied by disruption is observed or new cell generation in the pancreas is accelerated, it can be confirmed that the agent is effective for treatment of diseases accompanied by disruption of pancreatic tissue.

Model animals showing diseases accompanied by pancreatic β cell disruption include a model in which mainly streptozotocin is injected into the abdominal cavity (*The Journal of Clinical Investigation*, 48,. 2129-2139 (1969)), a model in which the pancreas is partially excised (*The Journal of Clinical Investigation*, 71, 1544-1553 (1983)) and the like. Animals which spontaneously onset diseases having a morbid state of pancreatic β cell disruption include a non-obese diabetic (NOD) mouse (*Exp*. *Animal,* 29, 1-13 (1980), BioBreeding (BB) rat (*Diabetes,* 31, Suppl. 1, 7-13 (1982)) and the like.

Improvement of symptoms of pancreatic β cell disruption can be evaluated by the measurement of concentrations of insulin, glucose and the like in the plasma or serum, the glucose tolerance at the time of glucose tolerance test, or the like. Furthermore, the improvement can also be determined by changes related to the diseases based on the body weight changes, amount of diet, urine sugar level measurement and the like.

The pancreatic cells differentiated inside the pancreas can be detected by the following method.

After irradiating a lethal dose of radiation in advance, a bone marrow chimeric mouse transplanted with a bone marrow derived from a transgenic mouse of the same strain capable of constitutively expressing green fluorescent protein (GFP) is prepared, and the pancreas of the bone marrow chimeric mouse is damaged by the above method, and then the agent for preventing and/or treating diseases accompanied by tissue disruption of the present invention is administered. The differentiated pancreatic cells can be identified based on fluorescence of GFP.

### (4) Kidney tissue

For example, according to the following method, it can be confirmed that the agent for preventing and/or treating diseases accompanied by tissue disruption of the present invention is useful for treatment of disease accompanied by disruption of kidney tissue.

The above agent for preventing and/or treating diseases accompanied by tissue disruption of the present invention is administered to a renal cell-disrupted model mouse, and if the alleviation of symptoms accompanied by disruption is observed or new cell generation in the kidney is accelerated, it can be confirmed that the agent is effective for treatment of diseases accompanied by disruption of kidney tissue. As the kidney disruption model, various glomerular nephritis models are preferred. Model animals which show a morbid state similar to that of glomerular nephritis include an anti-Thy-1 glomerular nephritis model which is induced by the intravenous injection of anti-Thy-1 antibody (*Kidney and Dialysis,* Special Issue of 1991, Renal Disease Models, published by Tokyo Igaku-sha), a Masugi glomerular nephritis model which is induced by an antibody for glomerular basement membrane (*Kidney and Dialysis,* Special Issue of 1991, Renal Disease Models, published by Tokyo Igaku-sha) and the like, and model animals which show a morbid state similar to that of renal insufficiency include a model in which bovine serum albumin (BSA) is injected into the abdominal cavity (*Kidney International,* 55, 890-898 (1999)).

Improvement of symptoms of renal cell disruption can be evaluated by measurement of a urinary protein excretion amount or the like.

The renal cell differentiated inside the kidney can be detected by the following method.

After irradiating a lethal dose of radiation in advance, a bone marrow chimeric mouse transplanted with a bone marrow derived from a transgenic mouse of the same strain capable of constitutively expressing green fluorescent protein (GFP) is prepared, and the kidney of the bone marrow chimeric mouse is damaged by the above method, and then the agent for preventing and/or treating diseases accompanied by tissue disruption of the present invention is administered. The differentiated renal cell can be identified based on fluorescence of GFP.

### (5) Lung tissue

For example, according to the following method, it can be confirmed that the agent for preventing and/or treating diseases accompanied by tissue disruption of the present invention is useful for treatment of diseases accompanied by disruption of lung tissue.

The above agent for preventing and/or treating diseases accompanied by tissue disruption of the present invention is administered to a lung cell-disrupted model mouse, and if the alleviation of symptoms accompanied by disruption is observed or new cell generation in the lungs is accelerated, it can be confirmed that the agent is effective for treatment of diseases accompanied by disruption of lung tissue. As the lung disruption model, a pulmonary emphysema-resembled model is preferred. Model animals which show a morbid state resembling pulmonary emphysema include a model in which a protease preparation mainly containing esterase is injected into the lung *(Environmental Research,* 33, 454-472 (1984)), a tobacco smoking model *(Chest,* 121, supplement, 188S-191S (2002)) and the like. Transgenic animals which show a morbid state of lung tissue disruption include a transgenic mouse of interleukin 13 (*The Journal of Clinical Investigation,* 106, 1081-1093 (2000)), a transgenic mouse of tumor necrosis factor-α (*American Journal of Physiology: Lung Cellular Molecular Physiology,* 280, L39-L49 (2001)) and the like.

Improvement of symptoms of lung cell disruption can be evaluated by analysis of tissue images of lung sections, measurement of wet weight and volume of the lung, or the like. Tissue images of lung sections can be analyzed by measurement of mean linear intercept length, alveolar area, or the like. The mean linear intercept length can be obtained by drawing a lattice of a predetermined length on a microscopic image of a lung section sample, measuring the distance between an alveolus wall crossing the linear line and an adjoining alveolus wall crossing the linear line, and calculating the average value. Furthermore, non-invasive measuring methods include X-ray photographing of the lungs, X-ray computed tomography (X-ray CT), magnetic resonance imaging (MRI) and the like. Although not directly, tissue disruption of the lungs can also be evaluated by changes related to the diseases based on measurement of body weight and respiratory function, measurement of the quantity of motion and moving distance within a predetermined period of time, exercise tolerance test, measurement of blood oxygen partial pressure, measurement of urinary and blood concentrations of desmosine which is an elastin degradation product of alveolus, or the like.

The cells differentiated in the lungs can be detected by the following method.

After irradiating a lethal dose of radiation in advance, a bone marrow chimeric mouse transplanted with a bone marrow derived from a transgenic mouse of the same strain capable of constitutively expressing green fluorescent protein (GFP) is prepared, and the lungs of the bone marrow chimeric mouse are damaged by the above method, and then the agent for preventing and/or treating diseases accompanied by tissue disruption of the present invention is administered. The differentiated lung cells can be identified based on fluorescence of GFP.

### (6) Skeletal muscle tissue

For example, according to the following method, it can be confirmed that the agent for preventing and/or treating diseases accompanied by tissue disruption of the present invention is useful for treatment of diseases accompanied by disruption of skeletal muscle tissue.

The above agent for preventing and/or treating diseases accompanied by tissue disruption of the present invention is administered to a skeletal muscle-disrupted model mouse, and if the alleviation of symptoms accompanied by disruption is observed or new cell generation in the skeletal muscle is accelerated, it can be confirmed that the agent is effective for treatment of diseases accompanied by disruption of skeletal muscle tissue. The model animals which show a morbid state resembling tissue disruption of the skeletal muscle include a model in which a muscle-toxic snake venom such as cardiotoxin or local anesthetic such as bupivacaine hydrochloride is mainly injected into skeletal muscle (*Igaku-no Ayumi (Advance in Medical Science),* 179, 276-280 (1996)) and the like. Transgenic animals having a morbid state of the disruption of skeletal muscle include a dystrophin-deficient mdx mouse (*Shinkei Shinpo (Advance in Nerves),* 45, 54-62 (2001)) and the like.

Improvement of symptoms of skeletal muscle tissue disruption can be evaluated by morphological observation based on the diameter and number of skeletal muscle fibers and fibrosis and fatty change of muscle. Furthermore, non-invasive measuring methods include muscle computed tomography (muscle CT), magnetic resonance imaging (MRI) and the like. Although not directly, the improvement can also be judged by changes related to the diseases based on measurement of serum creatine kinase (CK) value or the like.

The cells differentiated in the skeletal muscle can be detected by the following method.

After irradiating a lethal dose of radiation in advance, a bone marrow chimeric mouse transplanted with a bone marrow derived from a transgenic mouse of the same strain capable of constitutively expressing green fluorescent protein (GFP) is prepared, and the skeletal muscle of the bone marrow chimeric mouse is damaged by the above method, and then the agent for preventing and/or treating diseases accompanied by tissue disruption of the present invention is administered. The differentiated and formed skeletal muscle cells can be identified based on fluorescence of GFP.

### (7) Skin tissue

For example, according to the following method, it can be confirmed that the agent for preventing and/or treating diseases accompanied by tissue disruption of the present invention is useful for treatment of diseases accompanied by disruption of skin tissue.

The above agent for preventing and/or treating diseases accompanied by tissue disruption of the present invention is administered to a skin-disrupted model mouse, and if the alleviation of symptoms accompanied by disruption is observed or new cell generation in the skin is accelerated, it can be confirmed that the agent is effective for treatment of diseases accompanied by disruption of skin tissue. The model animals showing a morbid state resembling disruption of the skin tissue include a skin wound model in which a whole skin layer-lacking wound, burn wound, ischemic ulcer (decubitus) or infected wound is artificially prepared mainly on intractable model animal such as a genetic diabetes mellitus mouse (db/db), a genetic obese mouse (ob/ob), a steroid-treated mouse or a hepatopathy rat [*Tanpakushitsu Kakusan Koso (Protein, Nucleic Acid, Enzyme),* 45, 1145-1151 (2000)] and the like.

Improvement of symptoms of the skin tissue injury can be evaluated by measurement of wound area, measurement of skin tearing tension, measurement of the amount of exudates, measurement of the number of infiltrated leukocytes, measurement of the quantity of angiogenesis, measurement of the number of cells and collagen production in the granulation, or the like.

The cells differentiated inside the skin can be detected by the following method.

After irradiating a lethal dose of radiation in advance, a bone marrow chimeric mouse transplanted with a bone marrow derived from a transgenic mouse of the same strain capable of constitutively expressing green fluorescent protein (GFP) is prepared, and the skin of the bone marrow chimeric mouse is damaged by the above method, and then the agent for preventing and/or treating diseases accompanied by tissue disruption of the present invention is administered. The differentiated skin cells can be identified based on fluorescence of GFP.

### 6. Method for evaluating medicament which mobilizes multipotent stem cells into peripheral blood

The medicament which mobilizes multipotent stem cells into peripheral blood of the present invention can be evaluated as follows.

Cells induced by the medicament are collected from a transgenic mouse of the same strain capable of constitutively expressing green fluorescent protein (GFP), or cells mobilized by the medicament are collected, and a gene capable of labeling cells such as green fluorescent protein (GFP) is introduced into the cells, and then the cells are intravenously or intraventricularly administered. The differentiated cells can be identified based on the fluorescence of GFP.

Effects of the preventive and/or therapeutic agent of the present invention are described below in detail based on Reference Examples and Test Examples.

### Reference Example 1

### Increase of mononuclear cells in peripheral blood by administration of polypeptide having G-CSF activity:

Nartograstim (trade name: Neu-up, manufactured by Kyowa Hakko Kogyo Co., Ltd.) was subcutaneously injected to three male SD (Sprague-Dawley) rats (Charles River Japan) of nine weeks age for consecutive five days in a dose of 100 µg/kg, and peripheral blood was collected from the abdominal aorta on the 6th day after commencement of the administration. The mononuclear cell fraction was concentrated by density gradient centrifugation using NycoPrep 1.077 Animal (manufactured by Axis-Shield), and the number of cells was measured. As a result, mononuclear cells were increased from (2.32±0.16)×10⁶ cells/ml (mean value±standard error) to (4.29±0.93)×10⁶ cells/ml (mean value±standard error). Stem cells were contained therein.

### Reference Example 2

### Property of cells mobilized to peripheral blood by administration of G-CSF:

### (1) Transplantation of cells mobilized to peripheral blood mobilized by administration of G-CSF to mice irradiated with X-ray

The property of cells mobilized to peripheral blood by administration of G-CSF was analyzed by transplanting the cells to mice.

Mice (F4 of C57BL/6 × 129 strain) of ten weeks age, in which GFP gene was integrated into all the cells in the body and GFP protein was expressed, were divided into a group F comprising 3 mice and a group G comprising 1 mouse, and the following agents were administered to each of them. Specifically, to the group F, 10 µg per day of G-CSF (manufactured by Kyowa Hakko Kogyo Co., Ltd.; trade name: Neu-Up) was subcutaneously injected to each mouse for consecutive five days. To the group G, 200 µl per day of PBS (phosphate buffered saline) (pH 7.4) (manufactured by Life Technologies) was subcutaneously injected to each mouse for consecutive five days.

On the next day of the final administration of the agent, each mouse of the groups F and G was anesthetized with diethyl ether, and peripheral blood was collected from ophthalmic vein, recovered in a tube in which heparin sodium (manufactured by Takeda Chemical Industries, Ltd.) was previously charged, and the recovered peripheral blood was passed through a 100-µm cell strainer (manufactured by Becton Dickinson). With regard to the mice in group G, femur was excised, muscles attached to the femur were cut off using scissors so that whole femur was exposed, and then both ends were cut by scissors, front end of injection needle being attached with a needle of 27G manufactured by Terumo and containing PBS was inserted into the cut end of the knee joint side of the femur, and bone marrow cells were collected by blowing the PBS into a test tube, and the collected bone marrow cells were passed through a 100-µm cell strainer (manufactured by Becton Dickinson).

The peripheral blood or the bone marrow cells thus collected was transplanted by infusing it into C57BL/6 mice from tail vein as follows.

Firstly, the C57BL/6 mice of eight weeks age (CLEA Japan) were prepared, and on the day before the injection from tail vein, they were previously irradiated with X-ray in a dose of 12 Gy using an X-ray irradiating apparatus (manufactured by Hitachi Medico). Those mice were divided into groups H, I, J and K. To each mouse in the group H were transplanted to its tail vein 300 µl of peripheral blood derived from the mouse in group F; to each mouse in the group I were transplanted to its tail vein 300 µl per mouse of peripheral blood derived from the mouse in group G; to each mouse in group J were transplanted to its tail vein 3.0×10⁶ of bone marrow cells derived from the mouse in group G; and the mice in group K were not subjected to transplantation.

As a result, in the groups I and K, all the mice were dead within 7 to 10 days from the transplantation while, in the groups H and J, the mice were alive even after eight weeks from the transplantation. However, in some of mice of the group J in which bone marrow was transplanted, abnormalities in appearance and behavior were observed, for example, hair came out and skin was sore and they walked with inclined head and walking was unnatural. In the group H in which peripheral blood of mice to which G-CSF was administered was transplanted, no abnormality in appearance and behavior was observed.

The result shows that stem cells having an ability of differentiating into tissues such as skin are mobilized to peripheral blood of mice by the administration of G-CSF.

### (2) Dissection of mice to which cells were transplanted

The mice of the group H obtained in (1) were dissected and subjected to perfusion and fixation, and each organ was excised.

Specifically, the mouse was anesthetized by intraperitoneal injection of Nembutal (manufactured by Dainippon Pharmaceutical Co., Ltd.), total body was wetted by 70% ethanol, skin of thigh was picked up, the area from knee to root of thigh was cut and opened by scissors to expose thigh artery and vein. They were ligatured by suture made of silk (manufactured by Natsume Seisakusho Co., Ltd.), and the thigh was cut from the end of the ligatured site. Shinbone was excised therefrom, muscle attached to the shinbone was removed by scissors to expose total shinbone, both ends thereof were cut with scissors, front end of injection needle being attached with a needle of 23G manufactured by Terumo and containing PBS was inserted into the cut end of the knee joint side of the shinbone, and bone marrow cells were collected by blowing the PBS into a test tube.

On the other hand, the mouse was subjected to laparotomy and thoracotomy to expose the heart, 25G of a needle for intravenous injection equipped with a wing manufactured by Terumo was inserted into left ventricle, right auricle was cut off, and 20 ml of PBS were flowed and perfused through the whole body. Blood overflowed from the heart at that time was collected, as a peripheral blood, in a tube in which 100 units of heparin sodium (manufactured by Takeda Chemical Industries, Ltd.) were dispensed.

After all PBS was flowed, 20 ml of a fixing solution [4% PFA (paraformaldehyde), PBS] were flowed by the same operation to fix.

Then, the lung was excised together with trachea, and a 20-ml syringe equipped with a catheter attached to a surflow indwelling needle (20 G) manufactured by Terumo containing an OCT (optimum cutting temperature) compound (manufactured by Miles) diluted to two-fold with PBS was inserted into the trachea to bind the front end of catheter and the trachea using a suture made of silk (manufactured by Natsume Seisakusho Co., Ltd.), and 10 ml of the OCT solution diluted to two-fold with PBS was injected into lung through the trachea. After the injection, the lung was cut into blocks of several mm square, embedded with an OCT compound and frozen with isopentane which was cooled with dry ice.

Other organs were also excised from the mouse, immersed in a fixing solution [4% PFA (paraformaldehyde), PBS] at 4°C for 2 hours, rinsed with PBS, immersed in a high sucrose solution [20% sucrose, PBS], allowed to stand at 4°C over night, cut into blocks of several mm square on the next day, embedded with an OCT compound and frozen with isopentane which was cooled with dry ice.

The tissue thus frozen was sliced into the thickness of 10 µm using a cryostat, adhered to a slide glass coated with APS (manufactured by Matsunami) and well dried to prepare frozen sections.

The peripheral blood cells prepared during the above-mentioned operation were diluted with the same amount of 0.9% NaCl, which was layered on 1.4 ml of Nyco Prep 1.077 Animal (manufactured by Daiichi Pure Chemicals Co., Ltd.) and centrifuged at room temperature for 30 minutes at 600 × g. A layer of the mononuclear cell suspension of the interface was recovered, mixed with 1 ml of PBS and centrifuged at room temperature for 15 minutes at 400 × g. The supernatant was removed, the precipitated mononuclear cells were suspended in 0.5 ml of PBS, and the rate of numbers of GFP-positive cells among the peripheral mononuclear cells was measured using a FACS Calibur (manufactured by Becton Dickinson). As a result, the rate of the GFP-positive cells was 90.3%.

As to bone marrow cells, rate of the GFP-positive cells was also measured using a FACS Calibur in the same manner, and 87.3% were occupied by the GFP-positive cells.

Frozen section prepared from each organ by excision was observed under Axiophot 2, a fluorescence microscope manufactured by Zeiss, and it was confirmed that many GFP-positive cells were present in nearly all organs of the body such as lung, heart, liver, brain, stomach, skin, small intestine, large intestine, skeletal muscle, pancreas, spleen, kidney and trachea. Especially in the brain, many GFP-positive cells were observed in the areas such as olfactory bulb and choroid plexus. The result shows that stem cells mobilized to peripheral blood of mouse by administration of G-CSF functions for repair of various tissues of the body.

### (3) Identification of the property of GFP-positive cells by immunostaining

The frozen sections prepared in (2) were stained with various antibodies as follows to investigate the property of the GFP-positive cells.

Firstly, tissue sections of various organs were subjected to immunostaining using an anti-cytokeratin antibody. Cytokeratin is a marker for epithelial cells.

The frozen section prepared by excision of the skin was put on a slide glass, and the slide glass was immersed in PBS for 5 minutes three times. After washing the slide glass, it was immersed for 15 minutes in Proteinase K (manufactured by Gibco BRL) diluted with PBS so as to make the final concentration 10 mg/ml. After washing with PBS once, it was subjected to reaction with a fixing solution [4% PFA (paraformaldehyde), PBS] at room temperature for 15 minutes. After washing with PBS twice, it was immersed in a blocking solution [10% porcine serum (manufactured by Dako), PBS] at room temperature for 1 hour, and subjected to reaction at room temperature for 1 hour with a solution in which a primary antibody [Monoclonal Mouse Anti-Human Cytokeratin, Clones AE1/AE3] (manufactured by Dako) was diluted to 50-fold with PBS containing 1.5% of porcine serum. After washing with PBS four times, it was subjected to reaction at room temperature for 1 hour with a solution in which the second antibody [Cy3-conjugated Affini Pure Goat Anti-Mouse IgG (H + L)] (manufacture by Seikagaku Corporation) was diluted to 800-fold with PBS containing 1.5% of porcine serum. After washing with PBS four times, Vectashield Mounting Medium with DAPI (manufactured by Vector Laboratories) was dropped into the section, sealed with a cover glass and observed under a fluorescence microscope Axiophot 2 manufactured by Zeiss. Since GFP-positive and cytokeratin-positive cells were observed, which shows that, in the cells of peripheral blood mobilized by G-CSF used for the transplantation, there were contained stem cells having an ability of being taken to the skin and differentiating into epithelial cells.

Frozen section of large intestine was similarly subjected to the staining using an anti-cytokeratin antibody and observed under a fluorescence microscope, and as a result, GFP-positive and cytokeratin-positive cells were observed. The result shows that, in the cells of peripheral blood mobilized by G-CSF used for the transplantation, there were contained stem cells having an ability of being taken to the large intestine and differentiating into epithelial cells.

Frozen section of small intestine was similarly subjected to the staining using an anti-cytokeratin antibody and an antibody to CD 45 which is a marker for blood cells. When it was observed under a fluorescence microscope, there were observed GFP-positive and CD 45-negative cells. The result shows that, in the cells of peripheral blood mobilized by G-CSF used for the transplantation, there were contained stem cells having an ability of being taken to the small intestine and differentiating into cells which are other than blood cells.

Then, various organs were subjected to immunostaining using an anti-CD 45 antibody.

A slide glass on which frozen section prepared by excision of lung was washed by immersion for 5 minutes into PBS three times, the slide glass was immersed at room temperature for 10 minutes in a Dako Biotin Blocking System (1) solution (manufactured by Dako), washed with PBS twice, immersed at room temperature for 10 minutes into a Dako Biotin Blocking System (2) solution (manufactured by Dako) and washed with PB S twice again.

Then, it was immersed at room temperature for 1 hour in a blocking solution [10% porcine serum (manufactured by Dako), PBS] and then subjected to reaction at room temperature for 1 hour with a solution in which the primary antibody [Biotin anti-mouse CD 45 (LCA, Ly 5)] (manufactured by BD Pharmingen) was diluted to 100-fold with PBS containing 1.5% porcine serum. After washing with PBS four times, it was subjected to reaction at room temperature for 1 hour with a solution in which the second antibody (streptavidin, Alexa Fluor 594 conjugate) (manufactured by Molecular Probe) was diluted with PBS containing 1.5% of porcine serum to the final concentration 5 µg/ml. After washing with PBS four times, a Vectashield Mounting Medium with DAPI (manufactured by Vector Laboratories) was dropped on the section, sealed with a cover glass and observed under a fluorescence microscope. As a result, GFP-positive and CD 45-negative cells were observed. The result shows that, in the cells of peripheral blood mobilized by G-CSF used for the transplantation, there were contained stem cells having an ability of being taken to the lung and differentiating into cells other than blood cells.

Frozen section of liver was similarly subjected to the staining using an anti-CD 45 antibody, and observed under a fluorescence microscope, and GFP-positive and CD 45-negative cells were observed. The result shows that, in the cells of peripheral blood mobilized by G-CSF used for the transplantation, there were contained stem cells having an ability of being taken to the liver and differentiating into cells other than blood cells.

Frozen sections of heart, brain, stomach, skin, small intestine, large intestine, skeletal muscle and pancreas were similarly subjected to the staining using an anti-CD 45 antibody. When they were observed under a fluorescence microscope, there were observed GFP-positive and CD 45-negative cells in each of the tissues. The result shows that, in the cells of peripheral blood mobilized by G-CSF used for the transplantation, there were contained stem cells having an ability of being taken to heart, brain, stomach, skin, small intestine, large intestine, skeletal muscle and pancreas and differentiating into cells other than blood cells.

### Test Example 1

### Therapeutic effect of polypeptide having G-CSF activity on intestinal epithelium in mouse irradiated with X-ray:

New generation of cells does not occur in intestinal epithelium, bone marrow and the like of a mouse irradiated with X-ray (*Nature Review Cancer,* 3, 117-129 (2003)). Accordingly, a mouse irradiated with X-ray was prepared as an intestinal epithelium-disrupted model to examine whether or not the cell mobilized into peripheral blood by nartograstim contributes to the repair.

### (1) Transplantation of cells mobilized to peripheral blood by nartograstim administration to mouse irradiated with X-ray

Each of mice (C57BL/6 × 129 strain) of eight weeks age, in which GFP gene was integrated into all the cells in the body and GFP protein was expressed, were subcutaneously injected with 10 µg of nartograstim every day for consecutive five days. As the nartograstim, Neu-up 100 (manufactured by Kyowa Hakko Kogyo Co., Ltd.), which was dissolved in PBS (phosphate buffered saline) (pH 7.4) (manufactured by Life Technologies) so as to make the concentration 100 µg/ml, was used.

On the next day after the final administration of the nartograstim, each mouse was anesthetized with diethyl ether, and peripheral blood was collected from ophthalmic vein, recovered in a tube in which heparin sodium (manufactured by Takeda Chemical Industries, Ltd.) was previously charged, and the recovered peripheral blood was passed through a 100-µm cell strainer (manufactured by Becton Dickinson).

The peripheral blood thus collected was transplanted by infusing it into the mice from tail vein as follows.

Firstly, the C57BL/6 mice of eight weeks age (CLEA Japan) were prepared, and on the day before the transplantation, they were previously irradiated with X-ray in a dose of 12 Gy using an X-ray irradiating apparatus (manufactured by Hitachi Medico). On the next day, to each mouse, 300 µl per mouse of collected peripheral blood was transplanted from its tail vein. Thereafter, the chimeric mice thus obtained were examined whether they survive for at least 4 weeks (hereinafter referred to as "peripheral blood chimeric mice").

### (2) New generation of peripheral blood-derived cells mobilized by a polypeptide having G-CSF activity in intestinal epithelial cells in peripheral blood chimeric mice

Each of the peripheral blood chimeric mice prepared in the item (1) was dissected and subjected to perfusion and fixation to excise the small intestine and large intestine. Specifically, each of the peripheral blood chimeric mice was anesthetized by intraperitoneal injection of Nembutal (manufactured by Dainippon Pharmaceutical), and the heart was exposed by opening the abdomen and chest, 25G of a needle for intravenous injection equipped with a wing manufactured by Terumo was inserted into left ventricle, right auricle was cut off, and 20 ml of PBS were flowed and perfused through the whole body. After all PBS was flowed, 20 ml of a fixing solution [4% PFA (paraformaldehyde), PBS] were flowed by the same operation to fix.

Thereafter, the small intestine and large intestine were excised from the fixed mouse, soaked in the fixing solution [4% PFA (paraformaldehyde), PBS] at 4°C for 2 hours, rinsed with PBS, soaked in a high sucrose solution (20% sucrose, PBS) at 4°C overnight, and on the next day cut into blocks of several mm square, embedded with an OCT compound and frozen with isopentane which was cooled with dry ice.

The tissue thus frozen was sliced into the thickness of 10 µm using a cryostat, adhered to a slide glass coated with APS (manufactured by Matsunami) and well dried to prepare frozen sections.

The frozen tissue sections thus prepared from the small intestine and large intestine were subjected to immunostaining using an anti-cytokeratin antibody. Cytokeratin is a marker for epithelial cells.

The slide glass on which the frozen section was put was immersed in PBS for 5 minutes three times. After washing the slide glass, it was immersed for 15 minutes in Proteinase K (manufactured by Gibco BRL) diluted with PBS so as to make the final concentration 10 mg/ml. After washing with PBS once, it was subjected to reaction with a fixing solution [4% PFA (paraformaldehyde), PBS] at room temperature for 15 minutes. After washing with PBS twice, it was immersed in a blocking solution [10% porcine serum (manufactured by Dako), PBS] at room temperature for 1 hour, and subjected to reaction at room temperature for 1 hour with a solution in which a primary antibody [Monoclonal Mouse Anti-Human Cytokeratin, Clones AE1/AE3 (manufactured by Dako)] was diluted to 50-fold with PBS containing 1.5% of porcine serum. After washing with PBS four times, it was subjected to reaction at room temperature for 1 hour with a solution in which the second antibody [Cy3-conjugated Affini Pure Goat Anti-Mouse IgG (H + L) (manufacture by Seikagaku Corporation)] was diluted to 800-fold with PBS containing 1.5% of porcine serum. After washing with PBS four times, Vectashield Mounting Medium with DAPI (manufactured by Vector Laboratories) was dropped into the section, sealed with a cover glass and observed under a fluorescence microscope Axiophot 2 manufactured by Zeiss. As a result, GFP-positive and cytokeratin-positive cells were observed. Accordingly, it was confirmed that, in the small intestine and large intestine disrupted by X-ray irradiation, epithelial cells of respective tissue were differentiated from the cells mobilized to peripheral blood by the nartograstim used in the transplantation.

### Test Example 2

### Therapeutic effect of polypeptide having G-CSF activity on the liver in carbon tetrachloride-administered model mouse:

Using a model mouse in which the liver had been disrupted by administration of carbon tetrachloride, whether or not cells mobilized to peripheral blood by nartograstim is differentiated into hepatocytes was examined as follows.

First, peripheral blood chimeric mice were prepared by injecting nartograstim-mobilized peripheral blood into mice previously irradiated with X-ray from tail vein in the same manner as in the item (1) of Test Example 1. Then, carbon tetrachloride (manufactured by Wako) was intraperitoneally injected to the peripheral blood chimeric mice thus prepared in a dose of 2 ml/kg. In this case, carbon tetrachloride was prepared by using mineral oil (manufactured by Sigma) as the solvent to adjust a volume ratio of carbon tetrachloride to mineral oil to 2:3.

Two weeks after the intraperitoneal injection, each of the mice was subjected to perfusion and fixation in the same manner as in the method shown in the item (2) of Test Example 1 to excise the liver. Thereafter, the liver was excised from the fixed mouse, soaked in the fixing solution [4% PFA (paraformaldehyde), PBS] at 4°C for 2 hours, rinsed with PBS, soaked in a high sucrose solution (20% sucrose, PBS) at 4°C overnight, and on the next day cut into blocks of several mm square, embedded with an OCT compound and frozen with isopentane which was cooled with dry ice.

The tissue thus frozen was sliced into the thickness of 10 µm using a cryostat, adhered to a slide glass coated with APS (manufactured by Matsunami) and well dried to prepare frozen sections.

The frozen tissue section thus prepared from the liver was subjected to immunostaining using an anti-albumin antibody. Albumin is a marker for hepatocytes.

The slide glass on which the frozen section prepared by excising the liver was put was immersed in PBS for 5 minutes three times. After washing the slide glass, it was immersed in a blocking solution [10% porcine serum (manufactured by Dako), PBS] at room temperature for 1 hour, and subjected to reaction at room temperature for 1 hour with a solution in which a primary antibody [Anti-mouse albumin rabbit polyclonal (manufactured by Dako)] was diluted to 200-fold with PBS containing 1.5% of porcine serum. After washing with PBS four times, it was subjected to reaction at room temperature for 1 hour with a solution in which the second antibody [Alexa Fluor 594-anti rabbit IgG (manufacture by Molecular Probe)] was diluted to 800-fold with PBS containing 1.5% of porcine serum. After washing with PBS four times, Vectashield Mounting Medium with DAPI (manufactured by Vector Laboratories) was dropped into the section, sealed with a cover glass and observed under a fluorescence microscope. As a result, GFP-positive and albumin-positive cells which were morphologically similar to hepatocytes were observed. Accordingly, it was confirmed that, in the liver disrupted by carbon tetrachloride, hepatocytes were differentiated from the cells mobilized to peripheral blood by the nartograstim.

### Test Example 3

### Therapeutic effect of polypeptide having G-CSF activity on skeletal muscle in cardiotoxin-administered model mouse:

Using a model mouse in which the skeletal muscle had been disrupted by administration of cardiotoxin, whether or not cells mobilized to peripheral blood by nartograstim is differentiated into skeletal muscle fibers was examined as follows.

First, peripheral blood chimeric mice were prepared by injecting nartograstim-mobilized peripheral blood into mice previously irradiated with X-ray from tail vein in the same manner as in the item (1) of Test Example 1. Cardiotoxin was intramuscularly injected to the anterior tibial muscle of the peripheral blood chimeric mice thus prepared in a dose of 25 to 50 µl. In this case, cardiotoxin (manufactured by Latoxan) was used by dissolving in PBS so as to make the concentration 1 µM.

Next, four weeks after the intramuscular injection, the anterior tibial muscle was excised from each of the mice, soaked in the fixing solution [4% PFA (paraformaldehyde), PBS] at 4°C for 2 hours, rinsed with PBS, soaked in a high sucrose solution (20% sucrose, PBS) at 4°C overnight, and on the next day cut into blocks of several mm square, embedded with an OCT compound and frozen with isopentane which was cooled with dry ice.

The tissue thus frozen was sliced into the thickness of 10 µm using a cryostat, adhered to a slide glass coated with APS (manufactured by Matsunami) and well dried to prepare frozen sections.

The frozen tissue section thus prepared from the anterior tibial muscle was subjected to immunostaining using an anti-Desmin antibody. Desmin is a marker for skeletal muscle fiber.

The slide glass on which the frozen section prepared by excising the anterior tibial muscle was put was immersed in PBS for 5 minutes three times. After washing the slide glass, it was immersed for 15 minutes in Proteinase K (manufactured by Gibco BRL) diluted with PBS so as to make the final concentration 10 mg/ml. After washing with PBS once, it was subjected to reaction with a fixing solution [4% PFA (paraformaldehyde), PBS] at room temperature for 15 minutes. After washing with PBS twice, it was immersed in a blocking solution [10% porcine serum (manufactured by Dako), PBS] at room temperature for 1 hour, and subjected to reaction at room temperature for 1 hour with a solution in which a primary antibody [Anti-Desmin Delipidized, Whole Antiserum D8281 (manufactured by Sigma)] was diluted to 40-fold with PBS containing 1.5% of porcine serum. After washing with PBS four times, it was subjected to reaction at room temperature for 1 hour with a solution in which the second antibody [Alexa Fluor 594-anti rabbit IgG (manufactured by Molecular Probe)] was diluted to 800-fold with PBS containing 1.5% of porcine serum. After washing with PBS four times, Vectashield Mounting Medium with DAPI (manufactured by Vector Laboratories) was dropped into the section, sealed with a cover glass and observed under a fluorescence microscope. As a result, GFP-positive and Desmin-positive skeletal muscle fibers which were morphologically judged to be skeletal muscle were observed. Accordingly, it was confirmed that, in the skeletal muscle tissue disrupted by cardiotoxin, skeletal muscle fibers were differentiated from the cells mobilized to peripheral blood by the nartograstim.

### Test Example 4

### Therapeutic effect of polypeptide having G-CSF activity on skin wound model mouse:

In accordance with a conventional method [*Biological & Pharmaceutical Bulletin* (*Biol. Pharm. Bull.*), 19, 530-535 (1996)], two female C57BL/KsJ-db/db Jcl mice of six weeks age (CLEA Japan) were anesthetized by intraperitoneal administration of Nembutal (manufactured by Dainippon Pharmaceutical), and after removing the dorsal side hair, all skin layer excision wounds were prepared by using a biopsy trepan of 4 mm in diameter (manufactured by Kai Industries).

Starting on the day of the excision wound preparation (hereinafter described as "0 day"), nartograstim was subcutaneously administered to one of the two mice in a dose of 10 µg/body once a day for consecutive 5 days. The other mouse was administered with PBS instead of nartograstim. The nartograstim (manufactured by Kyowa Hakko Kogyo Co., Ltd.) dissolved in PBS so as to make the concentration 100 µg/ml was used as nartograstim. After the preparation of the excision wound, the wound area was periodically measured to calculate the degree of excision wound healing. The wound area was measured by photographing the two excision wounds prepared on the central region of the dorsal side with a digital camera (Nikon COOLPIX 990) and then using an image analyzing software (NIH Image). In addition to the wound area (mm²), the ratio of wound area when wound area on the 0 day is defined as 100% is shown in Table 2 as wound area ratio (%).

**Table 2**

| | Days after preparation of excision wound | | | |
|---|---|---|---|---|
| | 0 day | 3 days | 7 days | 10 days |
| Nartograstim administration | 22.4 mm² (100%) | 17.0 mm² (75.9%) | 3.3 mm² (14.7%) | 0.7 mm² (3.1%) |
| | 17.3 mm² (100%) | 9.6 mm² (53.5%) | 3.0 mm² (17.3%) | 2.8 mm² (16.2%) |
| PBS administration | 13.2 mm² (100%) | 10.7 mm² (81.1%) | 6.4 mm² (48.5%) | 5.2 mm² (39.4%) |
| | 10.5 mm² (100%) | 7.8 mm² (74.3%) | 7.8 mm² (74.3%) | 5.1 mm² (48.6%) |

| | | | | |
|---|---|---|---|---|
| Wound area (mm²) | | | | |
| (Wound area ratio (%)) | | | | |

As is shown in Table 2, acceleration of the reduction of wound area and wound area ratio, namely skin regeneration accelerating effect, was found in the nartograstim-administered mice when compared with the PBS-administered mice.

### Test Example 5

### Evaluation by rat alveolus disruption model (1):

Swine pancreas elastase (hereinafter referred to as "elastase", specific activity 135 units/mg protein, manufactured by Elastin Products) was diluted with saline (manufactured by Otsuka Pharmaceutical) to 70 units/ml, and 500 µl thereof was endotracheally administered to each of male SD rats of nine weeks age (Charles River Japan). One unit of elastase has the activity to degrade 1 mg of elastin within 20 minutes at pH 8.8 and 37°C. Two weeks thereafter, they were divided into groups comprising 10 mice per group, in such a manner that the mean body weight of each group became almost the same. Filgrastim to be used was prepared by dissolving Gran Injection M 300 (manufactured by Kirin Brewery) in saline so as to make the concentration 20 µg/ml. In the filgrastim-administered group, three weeks after the administration of elastase, filgrastim was subcutaneously administered in a dose of 100 µg/kg once a day repeatedly for consecutive 5 days. In the elastase-administered group, nothing was administered after the elastase administration. In the saline-administered group, saline was administered instead of elastase, and nothing was administered thereafter. Five weeks after the elastase administration, the lungs were excised and fixed by injecting formalin via the respiratory tract under a pressure of 25 cm H₂O, and then the disruption degree of alveolus was measured by using sections. The mean linear intercept length was calculated by drawing 5 lines of 1.325 µm lattice, lengthwise and breadthwise respectively, on a microphotograph of lung section, measuring the alveolus wall crossing on the lines, and then dividing the total length of lattice lines by the number of crossed alveolus walls *(Environmental Research,* 42, 340-352 (1987)).

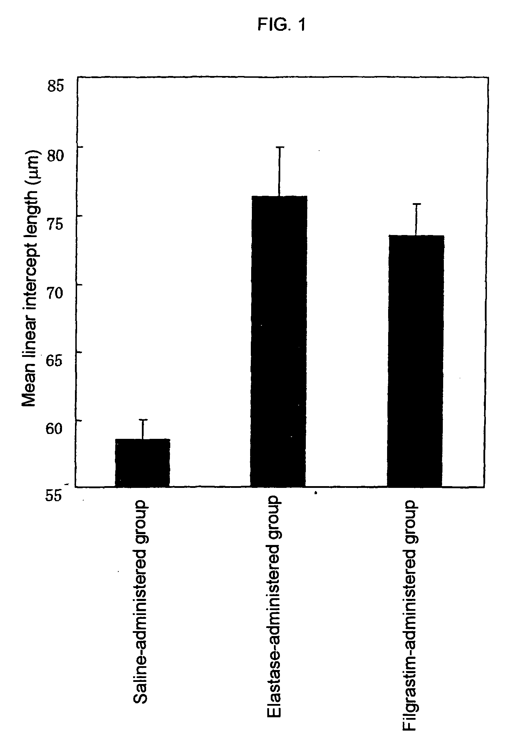

Fig. 1 shows the mean linear intercept length in terms of mean value ± standard error (SE). As shown in Fig. 1, lengthening of mean linear intercept length, namely disruption of alveolus walls, was observed by the administration of elastase. In the filgrastim-administered group, 16% of the disruption of alveolus walls was recovered.

### Test Example 6

### Evaluation by rat alveolus disruption model (2):

In the same manner as in Test Example 5, nartograstim was administered to rats in which alveoli were disrupted by the elastase treatment, and changes in the alveolus disruption were measured.

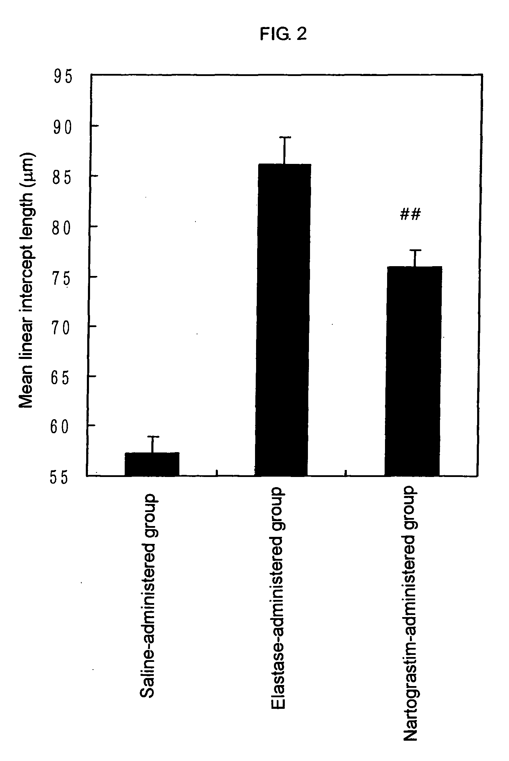

Nartograstim to be used was prepared by dissolving Neu-up 250 (manufactured by Kyowa Hakko Kogyo Co., Ltd.) in saline so as to make the concentration 40 µg/ml. Three weeks after the administration of elastase, nartograstim was subcutaneously administered in a dose of 200 µg/kg once a day for 5 consecutive days, followed by three times a week for 5 weeks. In the elastase-administered group, nothing was administered after the elastase administration. In the saline-administered group, saline was administered instead of elastase, and nothing was administered thereafter. Eight weeks after the elastase administration, the lungs were excised and analyzed in accordance with the method of Test Example 5. Fig. 2 shows the mean linear intercept length in terms of mean value ± standard error (SE).

As is shown in Fig. 2, lengthening of mean linear intercept length, namely disruption of alveolus walls, was observed by the administration of elastase. In the nartograstim-administered group, 35% of the disruption of alveolus walls was recovered.

### Test Example 7

### Evaluation by rat alveolus disruption model (3):

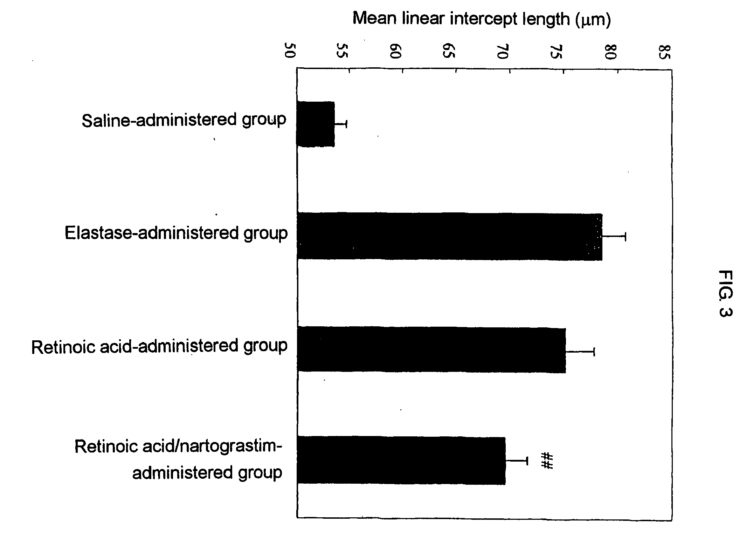

In the same manner as in Test Example 5, rats in which alveoli were disrupted by elastase treatment were administered with all trans-retinoic acid (hereinafter referred to as "retinoic acid"), or nartograstim and retinoic acid, and changes in the alveoli disruption were measured. Retinoic acid to be used was prepared by suspending retinoic acid (manufactured by Sigma Aldrich) in corn oil (manufactured by Wako Pure Chemical Industries) so as to make the concentration 3 mg/ml. Nartograstim to be used was prepared by dissolving Neu-up 100 (manufactured by Kyowa Hakko Kogyo Co., Ltd.) in saline so as to make the concentration 20 µg/ml. Three weeks after the administration of elastase, Nartograstim was subcutaneously administered in a dose of 100 µg/kg once a day for consecutive 5 days. Three weeks after the administration of elastase, retinoic acid was orally administered in a dose of 3 mg/kg once a day for consecutive 3 weeks. A group in which retinoic acid alone was administered (retinoic acid-administered group) and a group in which both of retinoic acid and nartograstim were administered (retinoic acid/nartograstim-administered group) were arranged. In the elastase-administered group, nothing was administered after the elastase administration. In the saline-administered group, saline was administered instead of elastase, and nothing was administered thereafter. Five weeks after the elastase administration, the lungs were excised and analyzed in accordance with the method of Test Example 5. Fig. 3 shows the mean linear intercept length in terms of mean value ± standard error (SE).

As is shown in Fig. 3, 14% disrupted alveolus walls were recovered in the retinoic acid-administered group. Strong recovery of disrupted alveolus walls was observed in the retinoic acid/nartograstim-administered group.

### Test Example 8

### Therapeutic effect of nartograstim upon db/db mouse:

db/db mouse is a db gene single recessive transgenic mouse, and known as a type II diabetes mellitus model mouse which spontaneously develops significant diabetic symptoms such as obesity, overeating and hyperinsulinism (*Joslin's Diabetes Mellitus*, pp.317-349 (1995)). Since obesity is caused in db/db mouse at around four to five weeks age after birth, and the blood sugar level increases in accordance with increase of body weight, it is considered that the hyperglycemic state induces tissue disruptions such as inflammation in organs of the whole body. Accordingly, using X-ray-irradiated db/db mouse, it was examined whether or not the cell mobilized to peripheral blood by nartograstim contributes to tissue repair.

### (1) Transplantation of bone marrow cells into X-ray-irradiated db/db mouse

First, bone marrow cells were transplanted prior to the transplantation of the cell mobilized to peripheral blood by nartograstim.

Female C57BL/KsJ-db/db mice of six weeks age (CLEA Japan) were used as the db/db mouse and irradiated with X-ray in a dose of 12 Gy using an X-ray irradiation apparatus (manufactured by Hitachi Medico) on the day before the transplantation. On the next day, into each of the mice, 3x106 cells isolated from the bone marrow of a mouse (C57BL/6 × 129 line) of eight weeks age, in which GFP gene was integrated into all the cells in the body and GFP protein was expressed, were transplanted from tail vein.

Four weeks after the transplantation, each of the mice was dissected and subjected to perfusion and fixation to excise each of the organs.

Specifically, the mouse was anesthetized by intraperitoneal injection of Nembutal (manufactured by Dainippon Pharmaceutical), and the heart was exposed by opening the abdomen and chest, 25G of a needle for intravenous injection equipped with a wing manufactured by Terumo was inserted into left ventricle, right auricle was cut off, and 20 ml of PBS were flowed and perfused through the whole body. After all PBS was flowed, 20 ml of a fixing solution [4% PFA (paraformaldehyde), PBS] were flowed by the same operation to fix.

Thereafter, each of the organs was excised from the fixed mouse, soaked in the fixing solution [4% PFA (paraformaldehyde), PBS] at 4°C for 2 hours, rinsed with PBS, soaked in a high sucrose solution (20% sucrose, PBS) at 4°C overnight, and on the next day cut into blocks of several mm square, embedded with an OCT compound and frozen with isopentane which was cooled with dry ice.

The tissue thus frozen was sliced into the thickness of 10 µm using a cryostat, adhered to a slide glass coated with APS (manufactured by Matsunami) and well dried to prepare frozen sections.

The frozen tissue sections thus prepared from each of the organs were subjected to immunostaining using various antibodies.

As a result, GFP-positive and albumin antibody-positive cells were observed in the section of the liver. Also, GFP-positive and insulin antibody-positive cells were observed in the section of the pancreas, and GFP-positive and sarcoma α-actin antibody-positive cells were observed in the section of the heart muscle. Furthermore, GFP-positive cells having a neuron-like shape and GFP-positive cells having a Purkinje cell-specific dendritic shape were observed in the section of the brain.

Thus, it was confirmed that, in the test regarding transplantation into db/db mouse irradiated with X-ray, hepatocytes, pancreatic β cells, heart muscle cells, neurons and Purkinje cells were differentiated from the bone marrow cells.

### (2) Transplantation of cells mobilized to peripheral blood by nartograstim administration, into db/db mouse irradiated with X-ray

Next, cells mobilized to peripheral blood by nartograstim administration were transplanted into db/db mouse irradiated with X-ray in the same manner as in the above item (1).

First, 10 µg of nartograstim was subcutaneously injected to each of mice (C57BL/6 × 129 strain) of eight weeks age, in which GFP gene was integrated into all the cells in the body and GFP protein was expressed, every day for consecutive five days. As the nartograstim, Neu-up 100 (manufactured by Kyowa Hakko Kogyo Co., Ltd.) which was dissolved in PBS (phosphate buffered saline) (pH 7.4) (manufactured by Life Technologies) so as to make the concentration 100 µg/ml was used.

On the next day of the final administration of the nartograstim, each mouse was anesthetized with diethyl ether, and peripheral blood was collected from ophthalmic vein, recovered in a tube in which heparin sodium (manufactured by Takeda Chemical Industries, Ltd.) was previously charged, and the recovered peripheral blood was passed through a 100-µm cell strainer (manufactured by Becton Dickinson).

The peripheral blood thus collected was transplanted by infusing it into the db/db mouse from tail vein as follows.

First, female C57BL/KsJ-db/db mice of six weeks age (CLEA Japan) as the db/db mouse to be subjected to transplantation were irradiated with X-ray in a dose of 9.5 Gy by using an X-ray irradiation apparatus (manufactured by Hitachi Medico) on the day before the transplantation. On the next day, the collected peripheral blood was transplanted into each of the mice via tail vein in a dose of 300 µl per animal.

Four weeks after the transplantation, each of the mice was dissected and subjected to perfusion and fixation to excise each of the organs, and the organs were embedded to prepare frozen sections.

The sections thus frozen of each of the organs were subjected to immunostaining with various antibodies.

As a result, GFP-positive and albumin antibody-positive cells were observed in the section of the liver. Also, GFP-positive and insulin antibody-positive cells were observed in the section of the pancreas, and GFP-positive and sarcoma α-actin antibody-positive cells were observed in the section of the heart muscle, and GFP-positive and cytokeratin antibody-positive cells were observed in the sections of the small intestine and the stomach. Furthermore, NeuN antibody-positive and GFP-positive cells having a neuron-like shape were observed in the section of the brain. Moreover, GFP-positive cells positioning at alveolus epithelial cells were observed in the section of the lungs.

Thus, it was confirmed that, in a test regarding transplantation into X-ray-irradiated db/db mouse, hepatocytes, pancreatic β cells, heart muscle cells, small intestine epithelial cells, stomach epithelial cells, neurons and alveolus epithelial cells were differentiated from the cells mobilized to peripheral blood by nartograstim.

### Test Example 9

### Effect of nartograstim on bone marrow chimera mouse:

Female C57BL/6 mice (CLEA Japan) of six weeks age and female C57BL/KsJ-db/db mice (CLEA Japan) of six weeks age were irradiated with X-ray in a dose of 9.5 Gy using an X-ray irradiation apparatus (manufactured by Hitachi Medical Corp.). On the next day, into each of the mice, 3×10⁶ cells isolated from the bone marrow of a male mouse (C57BL/6 × 129 line) of six to eight weeks age, in which GFP protein was expressed, were transplanted from tail vein.

The mice were divided into a nartograstim-administered group and a control group, and subjected to the following treatments. To the nartograstim-administered group, 7 days after the transplantation, 10 µg per day of nartograstim was subcutaneously injected to each mouse for consecutive 14 days. As the nartograstim, Neu-up (manufactured by Kyowa Hakko Kogyo Co., Ltd.) which was dissolved in PBS so as to make the concentration 100 µg/ml was used. Also, to the control group, 7 days after the transplantation, PBS was administered instead of the nartograstim for consecutive 14 days.

One month after the bone marrow transplantation, each of the mice was dissected and subjected to perfusion and fixation to excise the liver, lungs and kidneys. Specifically, each of the peripheral blood chimeric mice was anesthetized by intraperitoneal injection of Nembutal (manufactured by Dainippon Pharmaceutical), and the heart was exposed by opening the abdomen and chest, 25G of a needle for intravenous injection equipped with a wing manufactured by Terumo was inserted into left ventricle, right auricle was cut off, and 20 ml of PBS were flowed and perfused through the whole body. Thereafter, 20 ml of a fixing solution [4% PFA (paraformaldehyde), PBS] were flowed by the same operation to fix. Thereafter, the lungs were excised with the trachea attached as it was, a 20-ml syringe equipped with a catheter attached to a Surflow custody needle (20G) (manufactured by Terumo) containing an OCT compound (manufactured Miles) diluted to two-fold with PBS was inserted into the trachea to bind the tip of the catheter to the trachea by using silk sutures (manufactured by Natsume Seisakusho), and 10 ml of the OCT solution diluted to 2-fold with PBS was injected into the lung via the trachea. After the injection, the lungs were cut into blocks of several mm square, embedded with an OCT compound and frozen with isopentane which was cooled with dry ice. The liver and kidneys were also excised from each of the mice, soaked in the fixing solution [4% PFA (paraformaldehyde), PBS] at 4°C for 2 hours, rinsed with PBS, soaked in a high sucrose solution (20% sucrose, PBS) at 4°C overnight, and on the next day cut into blocks of several mm square, embedded with an OCT compound and frozen with isopentane which was cooled with dry ice. The tissues thus frozen were sliced into the thickness of 10 µm using a cryostat, adhered to a slide glass coated with APS (manufactured by Matsunami) and well dried to prepare frozen sections.

Among the frozen tissue sections thus prepared, the section of the liver was subjected to immunostaining using an anti-albumin antibody. Albumin is a marker for hepatocytes.

The slide glass on which the frozen section prepared by excising the liver was put was immersed in PBS for 5 minutes three times. After washing the slide glass, it was immersed in a blocking solution [10% porcine serum (manufactured by Dako), PBS] at room temperature for 1 hour, and subjected to reaction at room temperature for 1 hour with a solution in which a primary antibody [Anti-mouse albumin rabbit polyclonal (manufactured by Dako)] was diluted to 200-fold with PBS containing 1.5% of porcine serum. After washing with PBS four times, it was subjected to reaction at room temperature for 1 hour with a solution in which the second antibody [Alexa Fluor 594-anti rabbit IgG (manufacture by Molecular Probe)] was diluted to 800-fold with PBS containing 1.5% of porcine serum. After washing with PBS four times, Vectashield Mounting Medium with DAPI (manufactured by Vector Laboratories) was dropped into the section, sealed with a cover glass and observed under a fluorescence microscope.

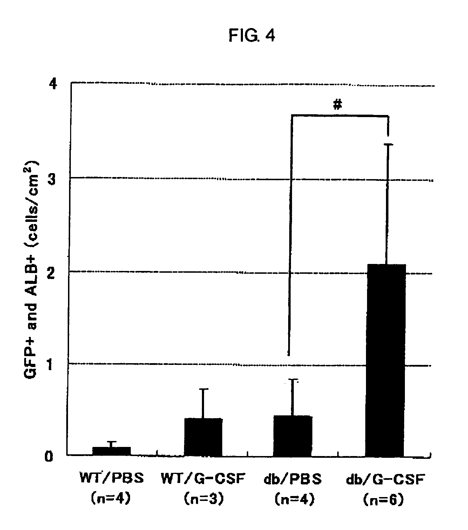

Thirty frozen sections were prepared from each mouse, and the number of GFP-positive and albumin-positive cells were counted. Fig. 4 shows the numbers of the GFP-positive and albumin-positive cells divided by unit area in terms of mean value ± standard error. As shown in Fig. 4, about 4.5-fold increase was confirmed for the nartograstim-administered C57BL/6 mice, and about 4.8-fold increase was confirmed for the nartograstim-administered C57BL/KsJ-db/db mice. Accordingly, bone marrow-derived cells differentiated to hepatocytes were increased by the administration of the nartograstim.

Furthermore, the numbers of GFP-positive cells were counted for the frozen sections of the lungs and kidneys. As a result, it was found that the numbers of GFP-positive cells were increased by the administration of nartograstim.

### BRIEF DESCRIPTION OF THE DRAWINGS

Fig. 1 shows change of the mean linear intercept length of alveoluses by administration of elastase and function by administration of filgrastim. The ordinate shows the mean linear intercept length (µm).
Fig. 2 shows change of the mean linear intercept length of alveoluses by administration of elastase and function by administration of nartograstim. The ordinate shows the mean linear intercept length (µm). ## represents P<0.01 (comparison with elastase-administered group, Wilcoxon rank sum test)
Fig. 3 shows change of the mean linear intercept length of alveoluses by administration of elastase and function by administration of retinoic acid or retinoic acid/nartograstim. The ordinate shows the mean linear intercept length (µm). ## represents P<0.01 (comparison with elastase-administered group, Wilcoxon rank sum test).
Fig. 4 shows the number of GFP- and Albumin-positive cells per unit area with or without nartograstim administration. The unit on the ordinate is cells/cm². # represents P<0.05 (Student's t-test). WT and db represent C57BL/6 mice group and C57BL/KsJ-db/db mice group, respectively.

### BEST MODE FOR CARRYING OUT THE INVENTION

The present invention is described below in detail based on Examples, but the present invention is not limited thereto.

### Example 1

### Injections (nartograstim):

According to the usual method, an injection having the following composition was prepared.

| | |
|---|---|
| Nartograstim | 25 µg |
| Polysorbate 80 | 2.5 g |
| Lactose | 5 mg |
| Saline | 0.5 ml |

### Example 2

### Injection (single agent of nartograstim and all- trans-retinoic acid):

According to the usual method, an injection having the following composition was prepared.

| | |
|---|---|
| Nartograstim | 25 µg |
| All-trans-retinoic acid | 1.0 mg |
| Polysorbate 80 | 2.5 g |
| Lactose | 5 mg |
| Saline | 0.5 ml |

### INDUSTRIAL APPLICABILITY

The present invention provides an agent for preventing and/or treating diseases accompanied by tissue disruption, which comprises a granulocyte colony-stimulating factor as an active ingredient.

SEQUENCE LISTING

<110> KYOWA HAKKO KOGYO CO., LTD.

<120>

<130> 11570WO1

<150> JP2003-139604
<151> 2003-05-16

<160> 1

<170> PatentIn Ver. 2.1

<210> 1
<211> 174
<212> PRT
<213> Homo sapiens

<400> 1
Thr Pro Leu Gly Pro Ala Ser Ser Leu Pro Gln Ser Phe Leu Leu Lys
  1               5                  10                  15
Cys Leu Glu Gln Val Arg Lys Ile Gln Gly Asp Gly Ala Ala Leu Gln
             20                  25                  30
Glu Lys Leu Cys Ala Thr Tyr Lys Leu Cys His Pro Glu Glu Leu Val

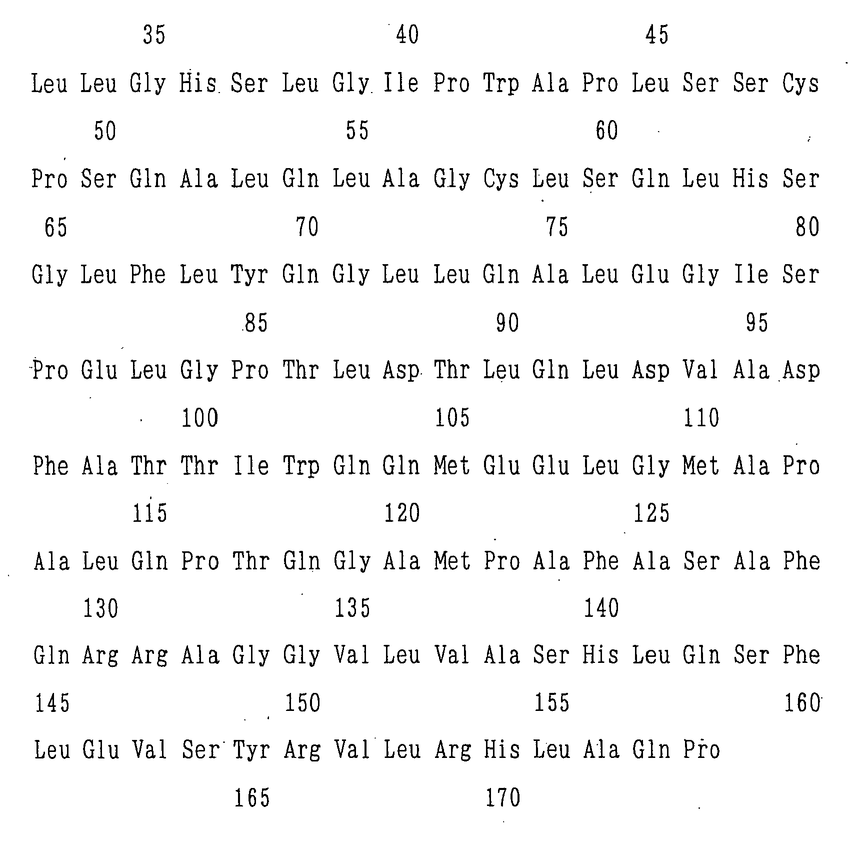

## Claims

1. An agent for preventing and/or treating diseases accompanied by tissue disruption, which comprises a polypeptide having granulocyte colony-stimulating factor activity as an active ingredient.

2. The agent according to claim 1, wherein the polypeptide comprises the amino acid sequence represented by SEQ ID NO:1.

3. The agent according to claim 1, wherein the polypeptide consists of an amino acid sequence in which at least one amino acid residue in the amino acid sequence represented by SEQ ID NO:1 is deleted, substituted and/or added, and has granulocyte colony-stimulating factor activity.

4. The agent according to claim 1, wherein the polypeptide consists of an amino acid sequence having a homology of 80% or more with the amino acid sequence represented by SEQ ID NO:1, and has granulocyte colony-stimulating factor activity.

5. The agent according to any one of claims 1 to 4, wherein the polypeptide is a chemically modified polypeptide having granulocyte colony-stimulating factor activity.

6. The agent according to claim 5, wherein the polypeptide is modified with polyalkylene glycol.

7. An agent for preventing and/or treating diseases accompanied by tissue disruption, which comprises (a) a polypeptide having granulocyte colony-stimulating factor activity and (b) retinoic acid or a retinoic acid derivative, wherein (a) and (b) are administered simultaneously, separately by keeping a period or as a single medicament comprising both of (a) and (b).

8. An agent for preventing and/or treating diseases accompanied by tissue disruption, which comprises (a) a polypeptide having granulocyte colony-stimulating factor activity and (b) a CXCR4 inhibitor, wherein (a) and (b) are administered simultaneously, separately by keeping a period or as a single medicament comprising both of (a) and (b).

9. The agent according to claim 8, wherein the CXCR4 inhibitor is AMD-3100 or a derivative thereof.

10. The agent according to any one of claims 1 to 9, wherein the disease accompanied by tissue disruption is selected from the group consisting of nervous diseases, circulatory organ system diseases, hepatic diseases, pancreatic diseases, digestive tract system diseases, renal diseases, skin diseases and lung diseases.

11. The agent according to claim 10, wherein the nervous disease is selected from the group consisting of cerebral infarction, cerebrovascular accidents, Parkinson disease, Alzheimer disease, Huntington chorea, spinal cord injury, depression and manic-depressive psychosis.

12. The agent according to claim 10, wherein the circulatory organ system disease is selected from the group consisting of obstructive vascular disease, myocardial infarction, cardiac failure and coronary artery disease.

13. The agent according to claim 10, wherein the hepatic disease is selected from the group consisting of hepatitis B, hepatitis C, alcoholic hepatitis, hepatic cirrhosis and hepatic insufficiency.

14. The agent according to claim 10, wherein the pancreatic disease is selected from the group consisting of diabetes mellitus and pancreatitis.

15. The agent according to claim 10, wherein the digestive tract system disease is selected from the group consisting of Crohn disease and ulcerative colitis.

16. The agent according to claim 10, wherein the renal disease is selected from the group consisting of IgA nephropathy, glomerular nephritis and renal insufficiency.

17. The agent according to claim 10, wherein the skin disease is selected from the group consisting of decubitus, burn injury, suture wound, lacerated wound, incision wound, bite wound, dermatitis, cicatricial keloid, keloid, diabetic ulcer, arterial ulcer and venous ulcer.

18. The agent according to claim 10, wherein the lung disease is selected from the group consisting of pulmonary emphysema, chronic bronchitis, chronic obstructive pulmonary disease, cystic fibrosis, sudden interstitial pneumonia (pulmonary fibrosis), diffuse pulmonary fibrosis, tuberculosis or asthma.

19. A medicament for mobilizing a multipotent stem cell from a tissue into peripheral blood, which comprises a polypeptide having granulocyte colony-stimulating factor activity as an active ingredient.

20. The medicament according to claim 19, wherein the polypeptide comprises the amino acid sequence represented by SEQ ID NO:1.

21. The medicament according to claim 19, wherein the polypeptide consists of an amino acid sequence in which at least one amino acid residue in the amino acid sequence represented by SEQ ID NO:1 is deleted, substituted and/or added, and has granulocyte colony-stimulating factor activity.

22. The medicament according to claim 19, wherein the polypeptide consists of an amino acid sequence having a homology of 80% or more with the amino acid sequence represented by SEQ ID NO:1, and has granulocyte colony-stimulating factor activity.

23. The medicament according to any one of claims 19 to 22, wherein the polypeptide is a chemically modified polypeptide having granulocyte colony-stimulating factor activity.

24. The medicament according to claim 23, wherein the polypeptide is modified with polyalkylene glycol.

25. A medicament for mobilizing a multipotent stem cell from a tissue into peripheral blood, which comprises (a) a polypeptide having granulocyte colony-stimulating factor activity and (b) retinoic acid or a retinoic acid derivative, wherein (a) and (b) are administered simultaneously, separately by keeping a period or as a single medicament comprising both of (a) and (b).

26. A medicament for mobilizing a multipotent stem cell from a tissue into peripheral blood, which comprises (a) a polypeptide having granulocyte colony-stimulating factor activity and (b) a CXCR4 inhibitor, wherein (a) and (b) are administered simultaneously, separately by keeping a period or as a single medicament comprising both of (a) and (b).

27. The medicament according to claim 26, wherein the CXCR4 inhibitor is AMD-3100 or a derivative thereof.

28. A method for preventing and/or treating diseases accompanied by tissue disruption, which comprises administering a polypeptide having granulocyte colony-stimulating factor activity.

29. A method for preventing and/or treating diseases accompanied by tissue disruption, which comprises administering (a) a polypeptide having granulocyte colony-stimulating factor activity and (b) retinoic acid or a retinoic acid derivative simultaneously or separately by keeping a period.

30. A method for preventing and/or treating diseases accompanied by tissue disruption, which comprises administering (a) a polypeptide having granulocyte colony-stimulating factor activity and (b) a CXCR4 inhibitor simultaneously or separately by keeping a period.

31. A method for mobilizing a multipotent stem cell from a tissue into peripheral blood, which comprises administering a polypeptide having granulocyte colony-stimulating factor activity.

32. A method for mobilizing a multipotent stem cell from a tissue into peripheral blood, which comprises administering (a) a polypeptide having granulocyte colony-stimulating factor activity and (b) retinoic acid or a retinoic acid derivative simultaneously or separately by keeping a period.

33. A method for mobilizing a multipotent stem cell from a tissue into peripheral blood, which comprises (a) a polypeptide having granulocyte colony-stimulating factor activity and (b) a CXCR4 inhibitor simultaneously or separately by keeping a period.

34. Use of a polypeptide having granulocyte colony-stimulating factor activity for the manufacture of an agent for preventing and/or treating diseases accompanied by tissue disruption.

35. Use of a polypeptide having granulocyte colony-stimulating factor activity for the manufacture of a medicament for mobilizing a multipotent stem cell from a tissue into peripheral blood.
